(19) **Europäisches Patentamt European Patent Office Office européen des brevets**

(11) **EP 4 333 649 B1**

(12) **EUROPEAN PATENT SPECIFICATION**

(45) Date of publication and mention
of the grant of the patent:
**23.04.2025  Bulletin 2025/17**

(21) Application number: **22727109.5**

(22) Date of filing: **03.05.2022**

(51) International Patent Classification (IPC):
*A23L 33/21* (2016.01)   *A23L 33/00* (2016.01)
*A61K 31/702* (2006.01)   *C12P 19/14* (2006.01)
*A61P 37/04* (2006.01)

(52) Cooperative Patent Classification (CPC):
**A23L 33/21; A23L 33/40; A61K 31/702;
A61P 37/04**

(86) International application number:
**PCT/EP2022/061824**

(87) International publication number:
**WO 2022/233855 (10.11.2022 Gazette 2022/45)**

(54) **GALACTOOLIGOSACCHARIDES FOR IMPROVING IMMUNE RESPONSE**

GALACTOOLIGOSACCHARIDE ZUR VERBESSERUNG DER IMMUNANTWORT

GALACTO-OLIGOSACCHARIDES POUR AMÉLIORER LA RÉPONSE IMMUNITAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.05.2021 EP 21171884**

(43) Date of publication of application:
**13.03.2024  Bulletin 2024/11**

(73) Proprietor: **N.V. Nutricia
2712 HM  Zoetermeer (NL)**

(72) Inventors:
• **POTAPPEL-VAN 'T LAND, Belinda
3584 CT Utrecht (NL)**
• **WIERTSEMA, Selma Paulien
3584 CT Utrecht (NL)**

(74) Representative: **Nederlandsch Octrooibureau
P.O. Box 29720
2502 LS The Hague (NL)**

(56) References cited:
EP-A1- 2 440 073      EP-B1- 2 440 073
WO-A1-2020/229690    US-A1- 2015 164 931
US-A1- 2017 209 472

• **SOHEIL VARASTEH ET AL: "Human milk
oligosaccharide 3'-galactosyllactose can protect
the intestinal barrier to challenges", JOURNAL
OF PEDIATRIC GASTROENTEROLOGY AND
NUTRITION, vol. 68, no. Suppl. 1, 15 May 2019
(2019-05-15), US, pages 1049 - 1050,
XP055711888, ISSN: 0277-2116, DOI: 10.1097/
MPG.0000000000002403**
• **AKBARI PEYMAN ET AL: "Galacto-
oligosaccharides Protect the Intestinal Barrier
by Maintaining the Tight Junction Network and
Modulating the Inflammatory Responses after a
Challenge with the Mycotoxin Deoxynivalenol in
Human Caco-2 Cell Monolayers and B6C3F1
Mice", THE JOURNAL OF NUTRITION, vol. 145,
no. 7, 27 May 2015 (2015-05-27), US, pages 1604 -
1613, XP055844764, ISSN: 0022-3166, DOI:
10.3945/jn.114.209486**
• **VANDENPLAS YVAN ET AL: "A Partly Fermented
Infant Formula with Postbiotics Including 3'-GL,
Specific Oligosaccharides, 2'-FL, and Milk Fat
Supports Adequate Growth, Is Safe and Well-
Tolerated in Healthy Term Infants: A Double-
Blind, Randomised, Controlled, Multi-Country
Trial", NUTRIENTS, vol. 12, no. 11, 20 November
2020 (2020-11-20), pages 3560, XP055844960,
DOI: 10.3390/nu12113560**

EP 4 333 649 B1

• CORRIER ET AL: "Mycotoxicosis: mechanisms of immunosuppression", VETERINARY IMMUNOLOGY AND IMMUNOPATHOLOGY, ELSEVIER BV, AMSTERDAM, NL, vol. 30, no. 1, 1 November 1991 (1991-11-01), pages 73 - 87, XP023795836, ISSN: 0165-2427, [retrieved on 19911101], DOI: 10.1016/0165-2427(91)90010-A

## Description

### Field of the invention

The present invention is in the field of infant and/or toddler nutrition. In particular the present invention relates to nutrition with galacto-oligosaccharides for use in counteracting the decreasing effect of food toxins, such as mycotoxins, on the immune response to pathogens or antigens.

### Background of the invention

Exposure of humans and animals to environmental factors such as food additives, drugs, industrial chemicals and bacterial and fungal metabolites, particularly early in life, can detrimentally affect health. The immune system is known to be most sensitive to chemical-induced toxicity. The mycotoxin deoxynivalenol (DON) is a highly prevalent food contaminant known to induce immunotoxicity in humans and animals. DON is produced as secondary metabolite from *Fusarium* fungi species, which contaminates human food at a global level, especially cereal and grain-based products. Acute and chronic exposure to DON can have a significant impact on growth and food consumption, and negatively affect intestinal, neurological and reproductive systems. The immune system is extremely sensitive to DON, since ingestion of very low levels can induce immunomodulatory effects.

In human milk, human milk oligosaccharides (HMOs) are present that provide health benefit to infants such as improvement of the intestinal microbiota, the intestinal gut barrier and the innate mucosal immune system. Non-digestible oligosaccharides such as galactooligosaccharides (GOS) comprising galactosyllactoses (GLs), such as 3'-GL, 4'-GL and 6'-GL, which are formed by the elongation of lactose with a further galactose residue, forming different galactosyl-lactoses can have similar functional properties as (HMOs). The composition of those GLs generated by trans-glycosylation highly depends on the enzyme source and technology chosen.

WO 2020/229690 shows that GOS/GLs have an anti-inflammatory properties on human intestinal epithelial cells *in vitro* and an GL-specific improved gut barrier effect.

WO 2013/172714 discloses a composition comprising a non-digestible oligosaccharide for use in the treatment, prevention or alleviation of a condition that has resulted from exposure to a trichothecene mycotoxin exposure in an individual, wherein the non-digestible oligosaccharide has a degree of polymerisation (DP) of 2 - 10.

WO 2016/013935 discloses a composition for use in reducing the risk of occurrence of or preventing whey protein allergy associated with mycotoxin exposure in an infant who consumes cereals or cereal-comprising products daily and suffers from an increased risk of whey protein allergy associated with mycotoxin exposure, said composition comprising non-digestible oligosaccharide. WO 2016/013935 is silent on any effect of DON on systemic and adaptive immune response to pathogens or a vaccine or antigen.

Varasteh et al., JPGN 2019, vol 68, suppl. 1, p 1049-1050 discloses a study directed toward the effect of beta1,3'-galactosyllactose toward gut barrier integrity in cell cultures exposed to DON. EP 2 440 073 concerns a nutritional composition comprising a combination of beta 1,3 galacto-oligosaccharides and beta 1,4 and/or 1,6 galacto-oligosaccharides and superior effects on immune system are demonstrated. US 2017/209472 is directed toward the use of a non-digestible oligosaccharide for providing nutrition to an infant suffering from an increased risk of food allergy. The infant is preferably at increased risk of mycotoxin exposure, for instance by consuming cereals. Akbari et al. J Nutr 2015, vol 145, p 1604-1613, relates to a study assessing the impact of GOS on DON-induced epithelial dysfunction of Caco-2 cells and in mice. US 2015/164931 discloses a composition comprising non-digestible oligosaccharide including GOS for treatment, prevention or alleviation of a mycotoxin exposure associated condition.

### Summary of the invention

The invention is as defined in the appended claims.

The mycotoxin deoxynivalenol (DON) is known to disrupt intestinal barrier and induce immunotoxicity Therefore, the effects of dietary interventions with trans-galactosyl-oligosaccharides (GOS) enriched in beta3'-GL (beta1,3'-galactosyllactose) on DON-induced immunotoxicity in a murine Influenza vaccination model was investigated. Mice received diets containing DON, GOS or a combination of these two.

DON exposure decreased Tbet[+] Th1 cells in the spleen and induced significant reduction in IFN-$\gamma$ secretion from splenocytes after *ex vivo* re-stimulation. Addition of GOS in DON-contaminated diets increased the frequency of Tbet[+] Th1 cells and secretion of IFN-$\gamma$ from re-stimulated splenocytes, and therefore prevent DON-induced reduction in type-1 immune responses in vaccinated animals. Moreover, consuming DON-contaminated diets caused a significant drop in frequency of B cells in the spleen of vaccinated mice, which corresponds to the reduction in vaccine-specific IgG production in these animals. Addition of GOS to the diet of DON-exposed mice restored the percentage of B cells to the values of the control group.

It thus was found that that supplementation with GOS comprising beta3'-GL could restore B cells in the spleen and improve vaccination responsiveness in DON-exposed mice.

## Detailed description of the invention

The invention concerns galacto-oligosaccharides or a nutritional composition comprising galacto-oligosaccharides for use in therapeutically preventing and/or ameliorating a food contaminant induced decrease of the B cell adaptive immune response to vaccination with an antigen that is derived from a virus, wherein the galacto-oligosaccharides comprise beta1,3'-galactosyllactose, and wherein the galacto-oligosaccharides comprise at least 20 % beta1,3'-galactosyllactose based on total weight of the galacto-oligosaccharides.

The nutritional composition is a synthetic nutritional composition.

According to the present invention, beta1,3'-galactosyllactose is seen as the active component for achieving the advantageous effect on prevention and/or amelioration of a food contaminant induced decrease of the B cell adaptive immune response to vaccination with an antigen that is derived from a virus.

Likewise the invention can be worded as beta1,3'-galactosyllactose for use in preventing and/or ameliorating a food contaminant induced decrease of the B cell adaptive immune response to vaccination with an antigen that is derived from a virus.

### *Food contaminant*

Mycotoxins are secondary metabolites produced by moulds and fungi contaminating cereal grains as well as forages, fruits, feed and food products as well as the environment (e.g., soil, water and air through aerosol acquired mycotoxicosis, etc.). Mycotoxins may have dangerous effects on human and animal health. Of particular note are the trichothecene mycotoxins, which are a class of compounds produced by the species *Fusarium graminearum.* This large family of sesquiterpene epoxides are closely related and vary by the position and number of hydroxylations and substitutions of a basic chemical structure. The major trichothecene produced by *Fusarium graminearum* is deoxynivalenol (DON) also known as vomitoxin for its ability to induce vomiting. The impact of DON on nutrient absorption in human intestinal epithelial cells has been investigated in Maresca et al. "The mycotoxin deoxynivalenol affects nutrient absorption in human intestinal epithelial cells" J. Nutr. Vol. 132 (2002) 2723 - 2731, and in Avantaggiato et al. "Evaluation of the intestinal absorption of deoxynivalenol and nivalenol by an in vitro gastrointestinal model, and the binding efficacy of activated carbon and other absorbent materials" Food and Chemical Toxicology vol. 42 (2004) 817 - 824.

Mycotoxins can appear in the food chain as a result of fungal infection of plant products (e.g., forage, grain, plant protein, processed grain by-products, roughage and molasses products), and can either be eaten directly by humans, or introduced by contaminated grains, livestock or other animal feedstuff(s). Since DON frequently occurs in toxicologically relevant concentrations in cereals and grains, it can be qualified as a genuine problem for all humans and animals consuming a diet comprising cereals and/or grains. It is a particular concern for infants, and with that in mind Codex Committees on Contaminants in Food (CCCF) have been dedicated to provide maximum limits for deoxynivalenol levels still deemed acceptable in raw cereal grains such as wheat and barley grain and infant formula. DON can also be present in the milk produced by lactating mothers consuming a diet contaminated with DON.

It was now found that upon consumption of diets contaminated with DON, a significant drop in frequency of B cells in the spleen of vaccinated mice was observed. The drop in frequency of B cells corresponded to the reduction in vaccine-specific IgG production. Addition of GOS that comprised beta1,3'-galactosyllactose to the diet of DON-exposed mice restored the percentage of B cells. Also DON contamination in the diet induced a reduction in Tbet$^+$ Th1 cells in the spleen of vaccinated mice. The addition of GOS that comprised beta1,3'-galactosyllactose to the diet increased the percentage of Tbet$^+$ Th1 cells in the spleen of DON-exposed mice and restored IFN-$\gamma$ production. Hence the use of GOS that comprise beta1,3'-galactosyllactose attenuate the adverse effects of DON on systemic adaptive immune response.

### *Beta1,3'-galactosyllactose*

The present invention relates to beta1,3'-galactosyllactose, herein also referred to as beta3'-GL, for use in preventing and/or ameliorating a food contaminant induced decrease of the B cell adaptive immune response to vaccination with an antigen that is derived from a virus.

This beta1,3'-galactosyllactose can be administered as such, in a suitable matrix, or in a nutritional composition. The beta1,3'-galactosyllactose may for example be part of a mixture of galacto-oligosaccharides (GOS), preferably beta-galacto-oligosaccharides (betaGOS). Beta1,3'-galactosyllactose is Gal-(beta 1,3)-Gal-(beta 1,4)-Glc, wherein Gal stands for galactose and Glc for glucose.

In a preferred embodiment, the beta1,3'-galactosyllactose is used as such. In another preferred embodiment, the beta1,3'-galactosyllactose is present in a nutritional composition. In one embodiment, the invention relates to galacto-oligosaccharides or to a nutritional composition comprising galacto-oligosaccharides for use in preventing and/or ameliorating a food contaminant induced decrease of the B cell adaptive immune response, wherein the galacto-oligosaccharides comprises beta1,3'-galactosyllactose, and wherein the galacto-oligosaccharides comprise at least 20 % beta1 ,3'-galactosyllactose based on total weight of the galacto-oligosaccharides, wherein the nutritional composition is a synthetic nutritional composition.

The nutritional composition comprising beta1,3'-galactosyllactose or the nutritional composition comprising galacto-oligosaccharides that comprise beta1,3'-galactosyllactose for use according to the invention are herein also referred to as the present nutritional composition, or nutritional composition according to the present invention, or final nutritional composition. The nutritional composition according to the present invention is not human milk.

As described above, the beta1,3'-galactosyllactose may be part of a mixture of galacto-oligosaccharides (GOS), preferably beta-galacto-oligosaccharides (betaGOS).

A suitable way to form GOS is to treat lactose with beta-galactosidases. Dependent on the specificity of the enzyme used, a galactose unit is hydrolysed from lactose and coupled to another lactose unit via a beta-linkage to form a trisaccharide. A galactose unit may also be coupled to another single galactose unit to form a disaccharide. Subsequent galactose units are coupled to form oligosaccharides. The majority of such formed oligosaccharides have a degree of polymerization (DP) of 7 or lower. Depending on the enzyme these linkages between the galactose residues can be predominantly beta1,4', beta1,6' or beta1,3'.

A suitable way to produce beta1,3'-galactosyllactose, is by using a beta-galactosidase from *S. thermophilus*. Particularly suitable is the use of beta-galactosidase from strain CNCM I-1470 and/or CNCM I-1620 in a process as disclosed in example 4 of FR2723960 or example 6 of EP0778885. *S. thermophilus* CNCM I-1620 was deposited under the Budapest Treaty on 23 August 1995 at Collection Nationale de Cultures de Microorganisms van Institute Pasteur, Paris, France by Compagnie Gervais Danone. Strain *S. thermophilus* CNCM I-1620 is also referred to as strain *S. thermophilus* ST065. *S. thermophilus* CNCM I-1470 was deposited under the Budapest Treaty on 25 August 1994 at Collection Nationale de Cultures de Microorganisms van Institute Pasteur, Paris, France by Compagnie Gervais Danone. Both strains have also been published in WO 96/06924. The composition of this GOS is also described in more detail in LeForestier et. al., 2009 Eur J Nutr, 48:457-464 and also in and in example 3 of WO 2020/229690. The amount of beta1,3'-galactosyllactose in this GOS preparation is in the range of 60-65 wt%, based on total galacto-oligosaccharides (excluding lactose, galactose and glucose). Another preferred sources of beta1,3'-galactosyllactose are commercially available GOS rich in beta1,3 and beta1,6 galacto-oligosaccharides include Bimuno from Clasado, or Purimune from GTC Nutrition. Beta1,6'- and beta1,3'-galactosyllactose can be enriched or purified from these GOS mixtures as known in the art, for example by size exclusion chromatography. Alternatively, pure beta1,3'-galactosyllactose is commercially available (Carbosynth Ltd, Compton, UK).

The GOS, including betaGOS, are non-digestible. Human digestive enzymes (including human lactase) are not able to hydrolyse GOS. GOS when consumed therefore reaches the large intestine intact and is available for fermentation by the intestinal microbiota.

Preferably the nutritional composition according to the present invention comprises at least 250 mg GOS per 100 ml, more preferably at least 400 even more preferably at least 600 mg per 100 ml. Preferably the composition does not comprise more than 2500 mg of GOS per 100 ml, preferably not more than 1500 mg, more preferably not more than 1000 mg. More preferably, the nutritional composition according to the present invention comprises GOS in an amount of 250 to 2500 mg/100 ml, even more preferably in an amount of 400 to 1500 mg/100ml, even more preferably in an amount of 600 to 1000 mg/100 ml.

In a preferred embodiment, the nutritional composition comprising galacto-oligosaccharides for use according to the present invention comprises at least 1 wt% galacto-oligosaccharides based on dry weight of the nutritional composition. Preferably the nutritional composition according to the present invention comprises at least 1.75 wt.% GOS based on dry weight of the nutritional composition, more preferably at least 2.8 wt.%, even more preferably at least 4.2 wt.%, all based on dry weight of the nutritional composition. Preferably the nutritional composition does not comprise more than 17.5 wt.% of GOS based on dry weight of the nutritional composition, more preferably not more than 10.5 wt.%, even more preferably not more than 7 wt%. The nutritional composition according to the present invention preferably comprises GOS in an amount of 1.75 to 17.5 wt.%, more preferably in an amount of 2.8 to 10.5 wt.%, most preferably in an amount of 4.2 to 7 wt.%, all based on dry weight of the nutritional composition.

Preferably the nutritional composition according to the present invention comprises at least 0.35 g GOS per 100 kcal, more preferably at least 0.6 g, even more preferably at least 0.8 g per 100 kcal. Preferably the composition does not comprise more than 3.7 g of GOS per 100 kcal, preferably not more than 2.5 g per 100 kcal, more preferably not more than 1.5 g per 100 kcal. More preferably, the nutritional composition according to the present invention comprises GOS in an amount of 0.35 to 3.7 g per 100 kcal, even more preferably in an amount of 0.6 to 2.5 g per 100ml, even more preferably in an amount of 0.8 to 1.5 g per 100 ml. Lower amounts result in a less effective composition, whereas the presence of higher amounts of GOS may result in side-effects such as osmotic disturbances, abdominal pain, bloating, gas formation and/or flatulence.

The nutritional composition comprising galacto-oligosaccharides for use according to the present invention comprises at least 20 % beta1 ,3'-galactosyllactose based on total weight of the galacto-oligosaccharides, preferably the nutritional composition comprising galacto-oligosaccharides comprises at least 25 % beta1 ,3'-galactosyllactose based on total weight of the galacto-oligosaccharides.

It is advantageous to have general GOS present in the present nutritional composition, besides the specific beta1 ,3'-galactosyllactose. A mixture of GOS with different sizes and linkages will have an increased beneficial effect on the microbiota and an improved production of short chain fatty acids, which in its turn have a further improved beneficial effect on immune function.

The total amount of GOS as defined for the present nutritional composition is including the amount of beta1,3'-galactosyllactose.

In a preferred embodiment, the nutritional composition according to the present invention comprises 0.25 to 2.5 g galacto-oligosaccharides per 100 ml, wherein 10 mg to 500 mg per 100 ml of the galacto-oligosaccharides is beta1,3'-galactosyllactose. In another preferred embodiment, the nutritional composition according to the present invention comprises 0.25 to 2.5 g galacto-oligosaccharides per 100 ml, wherein the amount of beta1,3'-galactosyllactose is more than 20 wt% based on total galacto-oligosaccharides, preferably more than 25 wt% based on total galacto-oligosaccharides. In another preferred embodiment, the nutritional composition according to the present invention comprises 0.25 to 2.5 g g galacto-oligosaccharides per 100 ml, wherein the amount of beta1,3'-galactosyllactose ranges from 10-500 mg per 100 ml. In another preferred embodiment, the nutritional composition according to the present invention comprises 0.25 to 2.5 g galacto-oligosaccharides per 100 ml, wherein the amount beta1,3'-galactosyllactose is more than 20 wt% based on total galacto-oligosaccharides, preferably more than 25 wt% based on total galacto-oligosaccharides, and wherein the amount of beta1,3'-galactosyllactose is between 150 mg and 250 mg per 100 ml.

In a preferred embodiment, the nutritional composition according to the present invention comprises 0.07 to 3.75 wt%, more preferably 0.1 to 2 wt.%, beta1,3'-galactosyllactose, based on dry weight of the nutritional composition. In a preferred embodiment, the nutritional composition comprises 0.07 to 0.375 wt% beta1,3'-galactosyllactose, based on dry weight of the nutritional composition. In another preferred embodiment, the nutritional composition comprises 1.125 to 1.725 wt% beta1,3'-galactosyllactose based on dry weight of the nutritional composition.

The nutritional composition according to the present invention preferably comprises 15 to 750 mg beta1,3'-galactosyllactose per 100 kcal of the nutritional composition. In a preferred embodiment, the nutritional composition comprises 15 to 75 mg beta1,3'-galactosyllactose per 100 kcal of the nutritional composition. In another preferred embodiment, the nutritional composition comprises 225 to 375 mg beta1,3'-galactosyllactose, per 100 kcal of the nutritional composition.

The nutritional composition according to the present invention preferably comprises 10 to 500 mg beta1,3'-galactosyllactose, per 100 ml of the nutritional composition. In a preferred embodiment, the nutritional composition comprises 10 to 75 mg, preferably 10 to 50 mg beta1,3'-galactosyllactose, per 100 ml of the nutritional composition. In another preferred embodiment, the nutritional composition comprises 150 to 250 mg beta1 ,3'-galactosyllactose per 100 ml of the nutritional composition.

Both when the beta1,3'-galactosyllactose is used as such and when it is present in a nutritional composition, it is preferred that the beta1,3'-galactosyllactose is administered in a daily dose of at least 0.075 g, preferably at least 0.10 g or 0.15 g. It is preferred that the maximum daily dose is 6 g, preferably 1.5 g, 1.3 g or 0.5 g. Preferably the beta1,3'-galactosyllactose is administered in a daily dose of 0.1 to 1.3 g, more preferred 0.1 g to 0.5 g.

It is preferred that the nutritional composition according to the present invention comprises 0.07 to 3.75 wt%, more preferably 0.1 to 2 wt.%, beta1,3'-galactosyllactose, based on dry weight of the nutritional composition and that the beta1,3'-galactosyllactose is administered in a daily dose of 0.10 to 6 g. Preferably the beta1,3'-galactosyllactose is administered in a daily dose of 0.1 to 1.3 g, more preferred 0.1 to 0.5 g.

Preferably the nutritional composition according to the present invention also comprises fructo-oligosaccharides (FOS), as described in more detail below.

Preferably the nutritional composition according to the present invention further comprises a source of protein, a source of lipids and a source of and digestible carbohydrates, as described in more detail below.

When the beta1,3'-galactosyllactose is present in a nutritional composition, in a preferred embodiment the nutritional composition is an infant formula, a follow on formula or a young child formula.

_Nutritional composition_

Preferably the beta1,3'-galactosyllactose is present in a nutritional composition. The nutritional composition according to the present invention is not human milk. The nutritional composition according to the present invention is not a natural milk, for example cow's milk. The nutritional composition is a synthetic nutritional composition.

The present nutritional composition is preferably enterally administered, more preferably orally.

The present nutritional composition is preferably an infant formula, a follow on formula or a young child formula.

Examples of a young child formula are toddler milk, toddler formula and growing up milk. More preferably the nutritional composition is a follow on formula or young child formula. An infant formula is defined as a formula for use in infants and can for example be a starter formula, intended for infants of 0 to 6 or 0 to 4 months of age. A follow on formula is intended for infants of 4 or 6 months to 12 months of age. At this age infants start weaning on other food. A young child formula, or toddler or growing up milk or formula is intended for children of above 12 months of age preferably up to 36 months of age. Preferably the present nutritional composition is a follow on formula or young child formula.

The present nutritional composition preferably comprises lipid, protein and carbohydrate and is preferably administered in liquid form. The present nutritional composition may also be in the form of a dry food, preferably in the form of a powder which is accompanied with instructions as to mix said dry food, preferably powder, with a suitable liquid, preferably water. The present nutritional composition may thus be in the form of a powder, suitable to reconstitute with water to provide a ready to drink nutritional composition, preferably a ready to drink infant formula, follow on formula or young child formula, more preferably a ready to drink follow on formula or young child formula. The nutritional composition according to the invention preferably comprises other fractions, such as vitamins, minerals, trace elements and other micronutrients in order to make it a complete nutritional composition. Preferably infant formulae comprise vitamins, minerals, trace elements and other micronutrients according to international directives.

The present nutritional composition preferably comprises lipid, protein and digestible carbohydrate wherein the lipid provides 25 to 65% of the total calories, the protein provides 6.5 to 16% of the total calories, and the digestible carbohydrate provides 20 to 80% of the total calories. Preferably, in the present nutritional composition the lipid provides 30 to 55% of the total calories, the protein provides 7 to 9% of the total calories, and the digestible carbohydrate provides 35 to 60% of the total calories. For calculation of the % of total calories for the protein, the total of energy provided by proteins, peptides and amino acids needs to be taken into account.

Preferably the lipid provides 3 to 7 g lipid per 100 kcal, preferably 3.5 to 6 g per 100 kcal, the protein provides 1.6 to 4 g per 100 kcal, preferably 1.7 to 2.3 g per 100 kcal and the digestible carbohydrate provides 5 to 20 g per 100 kcal, preferably 8 to 15 g per 100 kcal of the nutritional composition. Preferably the present nutritional composition comprises lipid providing 3.5 to 6 g per 100 kcal, protein providing 1.7 to 2.3 g per 100 kcal and digestible carbohydrate providing 8 to 15 g per 100 kcal of the nutritional composition.

Preferably the lipid provides 2.5 to 6.5 g lipid per 100 ml, preferably 2.5 to 4 g per 100 ml, the protein provides 1 to 3 g per 100 ml, preferably 1 to 1.5 g per 100 ml and the digestible carbohydrate provides 3 to 13 g per 100 ml, preferably 5 to 10 g per 100 ml of the nutritional composition. Preferably the present nutritional composition comprises lipid providing 2.0 to 6.5 g per 100 ml, protein providing 1 to 3 g per 100 ml and digestible carbohydrate providing 5 to 10 g per 100 ml of the nutritional composition.

Preferably the lipid provides 15 to 45 wt%, preferably 20 to 30 wt.%, based on dry weight of the composition, the protein provides 8 to 20 wt%, preferably 8.5 to 11.5 wt.%, based on dry weight of the composition and the digestible carbohydrates comprise 25 to 90 wt%, preferably 40 to 75 wt.%, based on dry weight of the composition. Preferably the present nutritional composition comprises lipid providing 20 to 30 wt.%, protein providing 8.5 to 11.5 wt.% and digestible carbohydrate providing 40 to 75 wt.%, all based on dry weight of the composition.

The present composition preferably comprises lipids. Preferably the present composition comprises at least one lipid selected from the group consisting of vegetable lipids. Preferably the present composition comprises a combination of vegetable lipids and at least one oil selected from the group consisting of fish oil, algae oil, fungal oil, and bacterial oil. Preferably the lipid comprises the essential fatty acids alpha-linolenic acid (ALA), linoleic acid (LA) and/or long chain polyunsaturated fatty acids (LC-PUFA). The LC-PUFA, LA and/or ALA may be provided as free fatty acids, in triglyceride form, in diglyceride form, in monoglyceride form, in phospholipid form, or as a mixture of one of more of the above. Preferably the present nutritional composition comprises at least one, preferably at least two lipid sources selected from the group consisting of rape seed oil (such as colza oil, low erucic acid rape seed oil and canola oil), high oleic sunflower oil, high oleic safflower oil, olive oil, marine oils, microbial oils, coconut oil, palm kernel oil.

The present nutritional composition preferably comprises long chain poly-unsaturated fatty acids (LC-PUFA). LC-PUFA are fatty acids or fatty acyl chains with a length of 20 to 24 carbon atoms, preferably 20 or 22 carbon atoms, comprising two or more unsaturated bonds. Preferably the composition comprises n3-LCPUFA. Preferably at least one, preferably two, LC-PUFA selected from docosahexaenoic acid (DHA), and eicosapentaenoic acid (EPA). These n3-LC-PUFA are considered to support immune function and may therefore be particularly advantageously combined with beta1,3'-galactosyllactose in order to support prevention or amelioration of a food contaminant induced decrease of the adaptive immune response. This combination has unexpected advantageous effects and preferably works synergistically. The LC-PUFA may be provided as free fatty acids, in triglyceride form, in diglyceride form, in monoglyceride form, in phospholipid form, or as a mixture of one of more of the above. Suitable sources of these LC-PUFA are e.g. fish oil and oil from Mortierella alpina.

The preferred content of LC-PUFA in the present nutritional composition does not exceed 15 wt.% of total fatty acids, preferably does not exceed 10 wt.%, even more preferably does not exceed 5 wt.%. Preferably the present composition comprises at least 0.2 wt.%, preferably at least 0.25 wt.%, more preferably at least 0.35 wt.%, even more preferably at least 0.5 wt.% LC-PUFA of total fatty acids, more preferably DHA.

The present nutritional composition preferably comprises protein. The protein used in the nutritional composition is preferably selected from the group consisting of non-human animal proteins, preferably milk proteins, vegetable proteins, such as preferably soy protein and/or rice protein, and mixtures thereof. The present nutritional composition preferably contains casein, and/or whey protein, more preferably bovine whey proteins and/or bovine casein. Thus in one embodiment the protein in the present nutritional composition comprises protein selected from the group consisting of whey protein and casein, preferably whey protein and casein, preferably the whey protein and/or casein is from cow's milk. Preferably the protein comprises less than 5 wt% based on total protein of free amino acids, dipeptides, tripeptides or hydrolysed protein. The present nutritional composition preferably comprises casein and whey proteins in a weight ratio casein : whey protein of 10 : 90 to 90 : 10, more preferably 20 : 80 to 80 : 20, even more preferably 35 : 65 to 55 : 45.

The wt% protein based on dry weight of the present nutritional composition is calculated according to the Kjeldahl-method by measuring total nitrogen and using a conversion factor of 6.38 in case of casein, or a conversion factor of 6.25 for other proteins than casein. The term 'protein' or 'protein component' as used in the present invention refers to the sum of proteins, peptides and free amino acids.

The present nutritional composition preferably comprises digestible carbohydrate. Preferred digestible carbohydrates are lactose, glucose, sucrose, fructose, galactose, maltose, starch and maltodextrin. Lactose is the main digestible carbohydrate present in human milk. The present nutritional composition preferably comprises lactose. Preferably the present nutritional composition does not comprise high amounts of carbohydrates other than lactose. Compared to digestible carbohydrates such as maltodextrin, sucrose, glucose, maltose and other digestible carbohydrates with a high glycemic index, lactose has a lower glycemic index and is therefore preferred. The present nutritional composition preferably comprises digestible carbohydrate, wherein at least 35 wt%, more preferably at least 50 wt%, more preferably at least 60 wt%, more preferably at least 75 wt%, even more preferably at least 90 wt%, most preferably at least 95 wt% of the digestible carbohydrate is lactose. Based on dry weight the present nutritional composition preferably comprises at least 25 wt% lactose, preferably at least 40 wt%, more preferably at least 50 wt% lactose.

The present nutritional composition preferably comprises non-digestible oligosaccharides (NDO). The term "oligosaccharides" as used herein refers to saccharides with a degree of polymerization (DP) of 2 to 250, preferably a DP 2 to 100, more preferably 2 to 60, even more preferably 2 to 10. If oligosaccharide with a DP of 2 to 100 is included in the present nutritional composition, this results in compositions that may contain oligosaccharides with a DP of 2 to 5, a DP of 50 to 70 and/or a DP of 7 to 60. The term "non-digestible oligosaccharides" (NDO) as used in the present invention refers to oligosaccharides which are not digested in the intestine by the action of acids or digestive enzymes present in the human upper digestive tract, e.g. small intestine and stomach, but which are preferably fermented by the human intestinal microbiota. For example, sucrose, lactose, maltose and maltodextrins are considered digestible.

Preferably the present non-digestible oligosaccharides are soluble. The term "soluble" as used herein, when having reference to a polysaccharides, fibres or oligosaccharides, means that the substance is at least soluble according to the method described by L. Prosky et al., J. Assoc. Off. Anal. Chem. 71, 1017-1023 (1988).

Beta1,3'-galactosyllactose is considered a non-digestible oligosaccharide, more in particular a non-digestible galacto-oligosaccharide. As described above, the beta1,3'-galactosyllactose may be present in the nutritional composition according to the invention as such, or as part of a mixture of galacto-oligosaccharides (GOS), preferably beta-galacto-oligosaccharides (BGOS). In a preferred embodiment the beta1,3'-galactosyllactose is present as part of a mixture of galacto-oligosaccharides.

Galacto-oligosaccharides, and preferred embodiments of the present nutritional composition comprising galacto-oligosaccharides, are described in more detail above.

Preferably the present nutritional composition also comprises fructo-oligosaccharides (FOS). The term "fructo-oligosaccharides" as used in the present invention refers to carbohydrates composed of over 50%, preferably over 65 % fructose units based on monomeric subunits, in which at least 50%, more preferably at least 75%, even more preferably at least 90%, of the fructose units are linked together via a beta-glycosidic linkage, preferably a beta-2,1 glycosidic linkage. A glucose unit may be present at the reducing end of the chain of fructose units. Preferably the fructo-oligosaccharides have a DP or average DP in the range of 2 to 250, more preferably 2 to 100, even more preferably 10 to 60. The term "fructo-oligosaccharides" comprises levan, hydrolysed levan, inulin, hydrolysed inulin, and synthesised fructo-oligosaccharides. Preferably the preparation comprises long chain fructo-oligosaccharides with an average DP above 20. Fructo-oligosaccharide suitable for use in the composition of the invention is also readily commercially available, e.g. RaftilineHP (Orafti). Preferably the nutritional composition according to the present invention comprises at least 25 mg FOS per 100 ml, more preferably at least 40 even more preferably at least 60 mg. Preferably the composition does not comprise more than 250 mg FOS per 100 ml, more preferably not more than 150 mg per 100 ml and most preferably not more than 100 mg per 100 ml. The amount of FOS is preferably 25 to 250 g fructo-oligosaccharides per 100 ml, preferably 40 to 150 g per 100 ml, more preferably 60 to 100 g per 100 ml. Preferably the nutritional composition according to the present invention comprises at least 0.15 wt.% FOS based on dry weight, more preferably at least 0.25 wt.%, even more preferably at least 0.4 wt.%. Preferably the composition does not comprise more than 1.5 wt.% FOS based on dry weight of the total composition, more preferably not more than 2 wt.%. The presence of FOS shows a further improved effect on the microbiota and its SCFA production. The presence of fructo-oligosaccharides with a DP or average DP in the range of 10 to 60 further improves the vaccination response. It is believed that fructo-oligosaccharides with a DP or average DP in the range of 10 to 60 acts synergistically with beta1,3'-galactosyllactose on improving vaccination response

Preferably the present nutritional composition comprises a mixture of galacto-oligosaccharides (including the beta1,3'-galactosyllactose) and fructo-oligosaccharides. Preferably the mixture of galacto-oligosaccharides and fructo-oligosaccharides is present in a weight ratio of from 1/99 to 99/1, more preferably from 1/19 to 19/1, more preferably from 1/1 to 19/1, more preferably from 2/1 to 15/1, more preferably from 5/1 to 12/1, even more preferably from 8/1 to 10/1, even more preferably in a ratio of about 9/1. This weight ratio is particularly advantageous when the galacto-oligosaccharides have a low average DP and fructo-oligosaccharides has a relatively high DP. Most preferred is a mixture of galacto-oligosaccharides with an average DP below 10, preferably below 6, and fructo-oligosaccharides with an average DP above 7, preferably above 11, even more preferably above 20.

The present nutritional composition preferably comprises 1.75 to 17.5 wt% total non-digestible oligosaccharides, more preferably 2.8 to 10.5 wt%, most preferably 4.2 to 7 wt%, based on dry weight of the nutritional composition. Based on 100 ml the present nutritional composition preferably comprises 0.25 to 2.5 g total non-digestible oligosaccharides, more preferably 0.4 to 1.5 g, most preferably 0.6 to 1 g, based on 100 ml of the nutritional composition. A lower amount of non-digestible oligosaccharides will be less effective in improving the gut barrier function, whereas a too high amount will result in side-effects of bloating and abdominal discomfort. The total amount of non-digestible oligosaccharides includes galacto-oligosaccharides, including beta1,3'-galactosyllactose, fructo-oligosaccharides and any additional non-digestible oligosaccharides that may further be present in the composition.

It is also important that the nutritional composition according to the present invention does not have an excessive caloric density, however still provides sufficient calories to feed the subject. Hence, the liquid food preferably has a caloric density between 0.1 and 2.5 kcal/ml, more preferably a caloric density of between 0.5 and 1.5 kcal/ml, even more preferably between 0.6 and 0.8 kcal/ml, and most preferably between 0.65 and 0.7 kcal/ml. The infant formula, follow on formula or young child formula according to the invention is for use in providing nutrition to an infant or young child, preferably an infant.

*Application*

It was found that galacto-oligosaccharides that comprise beta1,3'-galactosyllactose unexpectedly were capable to attenuate the adverse effects of deoxynivalenol (DON) on systemic adaptive immune response. Hence galacto-oligosaccharides that comprise beta1,3'-galactosyllactose or a nutritional composition comprising galacto-oligosaccharides that comprise beta1,3'-galactosyllactose can be used in preventing and/or ameliorating a food contaminant induced decrease of the B cell adaptive immune response to vaccination with an antigen that is derived from a virus.

The present invention thus relates to galacto-oligosaccharides or a nutritional composition comprising galacto-oligosaccharides for use in preventing and/or ameliorating a food contaminant induced decrease of the B cell adaptive immune response to vaccination with an antigen that is derived from a virus, wherein the galacto-oligosaccharides comprise beta1,3'-galactosyllactose, and wherein the galacto-oligosaccharides comprise at least 20 % beta1,3'-galactosyllactose based on total weight of the galacto-oligosaccharides, wherein the nutritional composition is a synthetic nutritional composition.

More in particular, it was found that galacto-oligosaccharides that comprise beta1,3'-galactosyllactose unexpectedly were capable to prevent the reduction in B cells due to the presence of the contaminant DON. Hence the adaptive immune response is immune response that is mediated via B cells.

Also more in particular it was found that galacto-oligosaccharides that comprise beta1,3'-galactosyllactose unexpectedly were capable to prevent the reduction of Tbet[+] Th1 cells in the spleen due to the presence of the contaminant DON and that galacto-oligosaccharides that comprise beta1,3'-galactosyllactose unexpectedly were capable to prevent DON-induced reduction in IFN-$\gamma$ production. Hence the B cell adaptive immune response is an immune response to an antigen that is derived from a virus.

In a preferred embodiment according to the invention, the decrease of the adaptive immune response is induced by a mycotoxin. In a preferred embodiment the mycotoxin is deoxynivalenol. In a preferred embodiment according to the invention, the decrease of the B cell adaptive immune response is induced by deoxynivalenol

In a preferred embodiment according to the invention, the galacto-oligosaccharides or nutritional composition comprising galacto-oligosaccharides is administered to an infant or young child, preferably a weaning infant or young child. In the context of the present invention, an infant is defined as a human having an age of 0 to 12 months and a young child is defined as a human having an age of 13 to 36 months. Weaning usually starts when an infant is about 4 to 6 months. The immune system of such infants and young children are especially vulnerable to food contaminants, as the adaptive immune system is still developing and levels of food contaminant have a larger impact because infants and young children have lower body weights and the daily doe based on body weight will be higher compared to adults. Therefore especially infants and young children will benefit from the invention.

In a preferred embodiment according to the invention, the galacto-oligosaccharides or nutritional composition comprising galacto-oligosaccharides is administered or consumed together or shortly before or after consumption of cereals or cereal-comprising products. Shortly before or after means preferably refers to a period of 4 hours, so the present nutritional composition is preferably consumed 4 hours or shorter before consumption of cereals or 4 hours or shorter after consumption of cereals or cereal-comprising products. Preferably the present nutritional composition is consumed 2 hours or shorter before consumption of cereals or cereal-comprising products or 2 hours or shorter after consumption of cereals or cereal-comprising products. Preferably the present nutritional composition is consumed 1 hour or shorter before consumption of cereals or cereal-comprising products or 1 hours or shorter after consumption of cereals or cereal-comprising products.

In a preferred embodiment, the nutritional composition for the use according to the invention is an infant formula, follow on formula or young child formula, preferably an follow on formula or young child formula. A young child formula is nutrition intended for children having an age of above 12 to 36 months. A follow on formula is nutrition intended for infants when weaning starts to 12 months. In a preferred embodiment, the methods or uses according to the present invention are for healthy infants.

In a further preferred embodiment, the galacto-oligosaccharides or nutritional composition comprising galacto-oligosaccharides for use according to the present invention are for subjects that are exposed to food contaminants, more preferably for infants or young children that are exposed to food contaminants. In yet a further preferred embodiment, the galacto-oligosaccharides or nutritional composition according to the present invention are for subjects that consume cereals or cereal-comprising products. In a further preferred embodiment, the galacto-oligosaccharides or nutritional composition according to the present invention are for infants that consume cereals or for young children that consume cereals or cereal-comprising products.

In the context of the present invention the term "prevention" means "reducing the risk of" or "reducing the severity of".

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

## EXAMPLES

*Example 1: Production of Galacto-oliqosaccharide (GOS) comprising beta3-'GL*

GOS comprising beta3'-GL was prepared using *S. thermophilus* ST065 (CNCM I-1620) beta galactosidase after which oligosaccharides were obtained by freeze drying. The dry powder contained beta3'-GL (22 g/100 g), lactose (45.5 g/100 g), glucose (15.6 g/100 g), galactose (5.1 g/100 g) and other oligosaccharides (11.7 g/100 g). This included mainly di-tri- and tetra-saccharides. More details on the GOS production and composition are given in example 4 of FR 2723960.

Table 1: Composition of the GOS powder.

|  | 100 g GOS (powder) | % based on GOS |
|---|---|---|
| lactose | 45.6 g | - |
| glucose | 15.6 g | - |
| galactose | 5.1 g | - |
| beta3'-GL | 22 g | 65 % |
| other non-digestible GOS not being beta3'-GL (DP2, DP3, DP4) | 11.7 | 35 % |
| total | 100 | 100 |

*Example 2: Dietary GOS rich in beta3'-GL improves vaccine-specific cellular and humoral responses in DON-exposed mice and increases Th1 cell activation and attenuated DON-induced modification in T cell populations in spleen.*

Materials and Methods:

Six-week-old female C57Bl/6JOlaHsd mice were purchased from Envigo (Horst, The Netherlands). Upon arrival, mice were conventionally housed under specific pathogen free (SPF) conditions with a light/dark cycle of 12 h/12 h (lights on from 7.00 am-7.00 pm) at controlled relative humidity (relative humidity of 50-55%) and temperature (21 ± 2°C) with access to food and tap water ad libitum. The animals were randomly grouped as 3 mice per cage in filter-topped makrolon cages (22 cm×16 cm×14 cm, floor area 350 cm2, Tecnilab- BMI , Someren, the Netherlands) with wood-chip bedding (Tecnilab- BMI , Someren in the Netherlands), and tissues (VWR, the Netherlands) were available as cage enrichment at the animal facility of Utrecht University. The animals received standard diets (pelleted food, AIN-93G, Ssniff Spezialdiäten, Soest, Germany) and routine care for a week upon arrival in the animal facility, before the start of the experiments. This study was conducted in accordance with institutional guidelines for the care and use of laboratory animals established by the Animal Ethics Committee of the Utrecht University, and all animal procedures related to the purpose of the research were approved under license of the national competent authority, securing full compliance the European Directive 2010/63/EU for the use of animals for scientific purposes.

Semi-purified AIN-93G soy protein-based diets were composed and mixed by different concentrations of DON (FERMENTEK Ltd, Jerusalem, Israel) and/or GOS comprising beta3'-GL of example 1 by Ssniff Spezialdiäten GmbH (Soest, Germany)

After a week of acclimatization, Animals were randomly divided into 4 groups (n=9 per group) and received either control or modified diets. 10 dietary groups were formed:

1) sham group (n=3) that was vaccinated with PBS and received standard rodent chow (AIN93G),
2) control group (n=9) that was vaccinated and received standard rodent chow,
3) group (n=9) that was vaccinated and received standard rodent chow with 12.5 mg/kg diet of DON
4) group (n=9) that was vaccinated and received standard rodent chow with 12.5 mg/kg diet of DON and 1 wt% GOS powder.

Vaccination was conducted 2 weeks after starting the diets, using Influvac (Abbott Biologicals B.V., Weesp, The Netherlands) from season 2015/2016 as previously described (Xiao et al, 2018, Front Immunol 9: article 452, https://doi.org/10.3389/fimmu.2018.00452). The mice received the primary and booster vaccinations by subcutaneous injections of 100 $\mu$l undiluted Influvac (containing hemagglutinin (HA) and neuraminidase antigens of three strains of myxovirus influenza, in a dose equivalent to 30 $\mu$g/mL HA per strain, in total 90 $\mu$g/ml HA). The booster vaccination was given 21 days after the primary vaccination. Sham group (n=3, negative control) which received injections of 100 $\mu$l PBS instead of vaccine was used to demonstrate the specificity of vaccine-induced response.

The animals were weighed before starting the diets (day -14) and before booster vaccination (day 21). The weight gain was calculated using the formula:

$$(weight\ on\ day\ 21) - (weight\ on\ day\ \text{-}14) = weight\ gain\ (g)$$

Blood was collected at the end of the experiment by orbital extraction under anesthesia and then animals were killed by cervical dislocation. Blood samples were centrifuged (10,000 rpm for 10 min) to collect the serum and were stored at -20°C until analysis. To determine serum concentration of vaccine-specific antibodies, enzyme-linked immunosorbent assay (ELISA) was performed. Serum samples were incubated in 96-well plates (Costar EIA/RIA plate, Alphen a/d Rijn, The Netherlands) pre-coated with 1:100 diluted Influvac in PBS. Final dilutions of 1:2000 and 1:8000 of serum samples were used for IgG1 and IgG2a measurements, respectively, and a dilution series of pooled serum that contained vaccine specific antibodies was added for standard curve calculation. For blocking nonspecific binding, the plate was incubated for 1 h with 2% BSA (Sigma, Zwijndrecht, The Netherlands) in PBS at room temperature. Anti-IgG1-biotin and anti-IgG2a-biotin (Becton Dickinson, Heerhugowaard, The Netherlands) antibodies were diluted 1:1000 in dilution buffer (PBS with 0.5% BSA and 0.1% Tween). The plates were subsequently incubated with a 1:20000 dilution of streptavidin-HRP (Biosource, Etten-Leur, The Netherlands) and optical density was measured with a Benchmark microplate reader (BioRad, Hercules, CA, USA) at a wavelength of 490 nm. Concentrations in test sera were calculated in arbitrary units (AU), relative to the standard curve.

Fresh splenocytes were isolated from spleens by crushing the tissue through 70 $\mu$m cell strainers on ice. After removing red blood cells by incubating in lysis buffer (8.3 g NH4Cl, 1 g KHC3O, and 37.2 mg EDTA dissolved in 1 L demi water and filter sterilized), splenocytes were counted and resuspended in RPMI 1640 medium containing 10% fetal bovine serum and penicillin (100 U/mL)/streptomycin (100 $\mu$g/mL) to reach the concentration of $10^7$ cell/ml. Cells were washed in PBS/1% BSA and incubated with anti-mouse CD16/CD32 (1:100 dilution in PBS/5% BSA; Mouse BD Fc Block, BD Pharmingen, San Jose, CA, USA) to block non-specific binding sites. For surface staining, cells were incubated at room temperature for 1 h with CD4- Brilliant Violet 510, CCR6-PE (BioLegend, San Diego, CA, United States), CD69-PE-Cy7, CXCR3-PE, CD25-PerCP-Cy5.5, (eBiosciences, Thermo Fisher Scientific, San Diego, CA, USA), T1ST2-FITC (MD Biosciences, St. Paul, MN, USA). Viable cells were distinguished by means of a fixable viability dye eFluor® 780 (eBioscience). For detecting intracellular transcription factors, cells were first fixed and permeabilized with Foxp3 Staining Buffer Set (eBioscience) according to manufacturer's protocol and then stained with Foxp3-FITC (eBioscience) and RoryT-Alexafluor 647 (BD Pharmingen, San Jose, CA, USA) antibodies. Results were collected with BD FACSCanto II flow cytometer (Becton Dickinson, Franklin Lakes, NJ, USA) and analyzed with FlowLogic software (Inivai Technologies, Mentone, VIC, Australia).

Bone marrow cells were isolated from femurs and tibias of healthy 11-week-old C57BL/6JOlaHsd mice. Collected cells were cultured in RPMI 1640 medium (Gibco) supplemented with 10% FBS and 100 U/mL penicillin/-streptomycin, 10 mM HEPES, 1 mM sodium pyruvate, and Eagles minimum essential medium (MEM) non-essential amino acids (all from Gibco Life Technologies) in the presence of 10 ng/mL GM-CSF (Prosepec, The Netherlands) for 6 days to obtain immature BMDC (iDC). Induced iDCs were then loaded with Influvac vaccine at a concentration of 0.9 $\mu$g/mL and incubated for 24 h at 37°C, 5% $CO_2$ to obtain matured DCs. iDCs treated with medium were used as negative control. Splenocytes collected from vaccinated mice were cocultured with matured DCs at 10:1 ratio, in 96-well U-bottom culture plates for 5 days at 37°C, 5% $CO_2$, with supplemented RPMI 1640 medium (Gibco).

Cell culture supernatants were collected at day 5 and stored at -20°C until use and analyzed for the concentration of interleukin (IL)-4, IL-6, IL-10, IL-13, tumor necrosis factor (TNF)-$\alpha$, macrophage inflammatory protein (MIP)-2 and interferon (IFN)-$\gamma$ using ProcartaPlex multiplex protein assay kit (Invitrogen, Thermo Fisher Scientific, Waltham, MA, USA) according to manufacturer's instructions.

Statistical Analysis

All data were analyzed by GraphPad Prism 8.0 software (GraphPad Software, San Diego, CA, USA) using one-way ANOVA, followed by a Bonferroni's multiple comparison post hoc test for selected comparisons. Data are presented as mean $\pm$ SEM. $*p < 0.05$, $**p < 0.01$ and $***p < 0.001$ were considered statistically significant.

Results:

There was no significant difference in average weight gain between different dietary groups. There was an antigen specific response to Influvac after i.d. injection, as determined by vaccine-specific immunoglobulin levels in serum.

The frequency and activation status of regulatory T cells (Treg) and T helper cells (Th1 and Th2) in isolated spleen samples were studied using flow cytometry. The percentage of $CD25^+$ $FoxP3^+$ Treg cells was not significantly affected by addition of DON in the diet of vaccinated mice, but addition of GOS to DON-contaminated diets significantly increased the percentage of Treg cells in the spleen of these animals as compared to DON-exposed groups.

No significant effect was observed on $CXCR3^+$ Th1 cells or $T1ST2^+$ Th2 cells due to the presence of GOS or DON in the diet.

DON contamination induced a reduction in $Tbet^+$ Th1 cells in the spleen of vaccinated mice, compared to control ($p < 0.01$), but surprisingly addition of GOS increased the percentage of $Tbet^+$ Th1 cells in the spleen of DON-exposed mice ($p < 0.05$), and restored it back to the values of the control group, see Table 2.

Table 2: Effects of DON and GOS comprising beta3'-GL on $Tbet^+$ Th1 cells (as % CXCR+CD4+ T cells)

| Diet | Mean (s.e.) | P value# |
|---|---|---|
| 1 Sham | 7.66 (0.25) | |
| 2 Control | 13.1 (1.04) | P < 0.05 vs group 1 |
| 3 12.5 DON | 8.06 (0.65) | P < 0.01 vs group 2 |
| 4 12.5 DON 1 % GOS | 11.86 (0.92) | P < 0.05 vs group 3 (p=0.1 vs group 1) |
| # One-way ANOVA followed by Bonferroni's post hoc test was used for selected groups. | | |

The frequency and activation status of B-cells in isolated spleen samples were studied using flow cytometry. Surface marker expression analysis of CD19 and CD220 revealed that DON induced significant the reduction in $CD19^+$ $B220^+$ B cell population ($p < 0.01$). Addition of GOS to DON-contaminated diets significantly ($p< 0.01$) increased the percentage of B cells in the spleen and was able to restore the effect of DON (See Table 3). CD27 expression was used to distinguish between memory and naive B cells.

Table 3: Effects of DON and GOS comprising 3'-betaGL on % B cells in the spleen

| Diet | Mean (s.e.) B cells in spleen | P value# |
|---|---|---|
| 1 Sham | 68.94 (2.34) | |
| 2 Control | 63.45 (1.63) | |
| 3 12.5 DON | 54.67 (1.63) | P < 0.01 compared to group 1. P < 0.05 vs group 2 |
| 4 12.5 DON 1 % GOS | 65.75 (2.59) | P <0.01 vs group 3 |
| # One-way ANOVA followed by Bonferroni's post hoc test was used for selected groups. | | |

In order to study cytokine production capacity of immune competent cells in vaccinated mice, collected splenocytes were re-stimulated *ex vivo* by co-culturing the cells with antigen-loaded dendritic cells. No significant effect for dietary GOS or DON was observed on concentrations of IL-4, IL-6, IL-13, TNF-$\alpha$ and MIP-2 in cell supernatants Dietary supplementation with GOS was able to prevent DON-induced reduction in IFN-$\gamma$ production (See Table 4).

Table 4: Effects of DON and GOS comprising beta3'-GL levels of IFN-gamma

| Diet | Mean s.e. IFN-gamma (pg/ml) | P value# |
|---|---|---|
| 2 Control | 55.7 (13.5) | |
| 3 12.5 DON | 9.57 (3.63) | P < 0.01 vs group 2 |
| 4 12.5 DON 1 % GOS | 34.95 (5.59) | |
| # One-way ANOVA followed by Bonferroni's post hoc test was used for selected groups. | | |

Adding DON in the diet of vaccinated mice had a specifically detrimental effect on B cell-mediated humoral immunity, as indicated by the reduced vaccine-specific immunoglobulin production. DON exposure also decreased Tbet[+] Th1 cells in the spleen and induced significant reduction in IFN-$\gamma$ secretion from splenocytes after *ex vivo* re-stimulation. Results of our study showed that addition of GOS in DON-contaminated diets increased the frequency of Tbet[+] Th1 cells and secretion of IFN-$\gamma$ from re-stimulated splenocytes, and therefore prevent DON-induced reduction in type-1 immune responses in vaccinated animals. Moreover, consuming DON-contaminated diets caused a significant drop in frequency of B cells in the spleen of vaccinated mice.

In conclusion, exposure to DON leads to modulated immune responses to vaccination. Dietary intervention with oligosaccharide mixture GOS comprising beta3'-GL attenuates the adverse effects of DON on the adaptive immune response.

*Example 3: Follow on Formula*

A follow on formula, provided as a powder in a pack with instructions to reconstitute with water to a ready to drink milk. When reconstituted the formula contains per 100 ml:

- 68 kcal
- about 1.4 g Protein (mainly whey protein and casein from bovine)
- about 3.2 g Lipid, wherein the amount of DHA is 0.52 wt%, EPA is 0.11 wt% and ARA is 0.52 wt% based on total fatty acids
- about 8.1 g digestible carbohydrates (mainly lactose)
- about 720 mg galacto-oligosaccharides, of which about 140 mg beta3'-GL, (500 mg from Vivinal GOS and 220 mg from GOS produced by the betagalactosidase from S. thermophilus CNCM-I-1620, respectively) and about 80 mg lcFOS (source RaftilinHP)

minerals, trace elements, vitamins and other micronutrients as known in the art and according to international directives for infant formula.

**Claims**

1. Galacto-oligosaccharides or nutritional composition comprising galacto-oligosaccharides for use in therapeutically preventing and/or ameliorating a food contaminant induced decrease of the B cell adaptive immune response to vaccination with an antigen that is derived from a virus, wherein the galacto-oligosaccharides comprise beta1,3'-galactosyllactose, and wherein the galacto-oligosaccharides comprise at least 20 % beta1,3'-galactosyllactose based on total weight of the galacto-oligosaccharides, wherein the nutritional composition is a synthetic nutritional composition.

2. The galacto-oligosaccharides or nutritional composition comprising galacto-oligosaccharides for use according to claim 1, wherein the food contaminant is a mycotoxin.

3. The galacto-oligosaccharides or nutritional composition comprising galacto-oligosaccharides for use according to claim 2, wherein mycotoxin is deoxynivalenol.

4. The galacto-oligosaccharides or nutritional composition comprising galacto-oligosaccharides for use according to any one of the preceding claims, wherein the galacto-oligosaccharides or nutritional composition comprising galacto-oligosaccharides is administered to an infant or young child, preferably a weaning infant or young child.

5. The galacto-oligosaccharides or nutritional composition comprising galacto-oligosaccharides for use according to any one of the preceding claims, wherein the galacto-oligosaccharides or nutritional composition comprising galacto-oligosaccharides is administered to a subject that consumes cereals or cereal-comprising products.

6. The galacto-oligosaccharides or nutritional composition comprising galacto-oligosaccharides for use according to any one of the preceding claims wherein administration or consumption is together or shortly before or after consumption of cereals or cereal-comprising products.

7. The nutritional composition comprising galacto-oligosaccharides for use according to any one of the preceding claims, wherein the nutritional composition comprises at least 1 wt% galacto-oligosaccharides based on dry weight of the nutritional composition.

8. The nutritional composition comprising galacto-oligosaccharides for use according to any one of the preceding claims, wherein the nutritional composition is an infant formula, a follow on formula or a young child formula, preferably a follow on formula or a young child formula.

9. Beta1,3'-galactosyllactose for use in therapeutically preventing and/or ameliorating a food contaminant induced decrease of the B cell adaptive immune response to vaccination with an antigen that is derived from a virus.


**Patentansprüche**

1. Galactooligosaccharide oder Nährstoffzusammensetzung umfassend Galacto-oligosaccharide zur Anwendung bei der therapeutischen Vorbeugung und/oder Linderung einer durch Lebensmittelverunreinigungen induzierten Abnahme der adaptiven B-Zell-Immunantwort auf eine Impfung mit einem Antigen, das von einem Virus stammt, wobei die Galactooligosaccharide Beta1,3'-Galactosyllactose umfassen und wobei die Galacto-oligosaccharide mindestens 20 % Beta1,3'-Galactosyllactose umfassen, basierend auf dem Gesamtgewicht der Galactooligosaccharide, wobei die Nährstoffzusammensetzung eine synthetische Nährstoffzusammensetzung ist.

2. Galactooligosaccharide oder Nährstoffzusammensetzung umfassend Galacto-oligosaccharide zur Anwendung nach Anspruch 1, wobei die Lebensmittelverunreinigung ein Mykotoxin ist.

3. Galactooligosaccharide oder Nährstoffzusammensetzung umfassend Galacto-oligosaccharide zur Anwendung nach Anspruch 2, wobei das Mykotoxin Deoxynivalenol ist.

4. Galactooligosaccharide oder Nährstoffzusammensetzung umfassend Galacto-oligosaccharide zur Anwendung nach einem der voranstehenden Ansprüche, wobei die Galactooligosaccharide oder die Nährstoffzusammensetzung umfassend Galactooligosaccharide an einen Säugling oder ein junges Kind, vorzugsweise einen entwöhnenden Säugling oder junges Kind, verabreicht wird.

**5.** Galactooligosaccharide oder Nährstoffzusammensetzung umfassend Galacto-oligosaccharide zur Anwendung nach einem der voranstehenden Ansprüche, wobei die Galactooligosaccharide oder Nährstoffzusammensetzung umfassend Galactooligosaccharide an ein Subjekt verabreicht wird, das Getreide oder Getreide umfassende Produkte verzehrt.

**6.** Galactooligosaccharide oder Nährstoffzusammensetzung umfassend Galacto-oligosaccharide zur Anwendung nach einem der voranstehenden Ansprüche, wobei die Verabreichung oder der Verzehr zusammen mit oder kurz vor oder nach dem Verzehr von Getreide oder Getreide umfassenden Produkten erfolgt.

**7.** Nährstoffzusammensetzung umfassend Galactooligosaccharide zur Anwendung nach einem der voranstehenden Ansprüche, wobei die Nährstoffzusammensetzung mindestens 1 Gew.-% Galactooligosaccharide auf Basis des Trockengewichts der Nährstoffzusammensetzung umfasst.

**8.** Nährstoffzusammensetzung umfassend Galactooligosaccharide zur Anwendung nach einem der voranstehenden Ansprüche, wobei die Nährstoffzusammensetzung eine Säuglingsanfangsnahrung, eine Folgenahrung oder eine Kleinkindnahrung, vorzugsweise eine Folgenahrung oder eine Kleinkindnahrung, ist.

**9.** Beta1,3'-Galactosyllactose zur Anwendung bei der therapeutischen Vorbeugung und/oder Linderung einer durch Lebensmittelverunreinigungen verursachten Abnahme der adaptiven B-Zellen-Immunantwort auf eine Impfung mit einem Antigen, das von einem Virus abstammt.


**Revendications**

**1.** Galacto-oligosaccharides ou composition nutritionnelle comprenant des galacto-oligosaccharides pour l'utilisation dans la prévention thérapeutique et/ou l'amélioration d'une diminution induite par un contaminant alimentaire de la réponse immunitaire adaptative des cellules B à la vaccination avec un antigène qui est dérivé d'un virus, où les galacto-oligosaccharides comprennent du bêta-1,3'-galactosyllactose, et où les galacto-oligosaccharides comprennent au moins 20 % de bêta-1,3'-galactosyllactose sur la base du poids total des galacto-oligosaccharides, où la composition nutritionnelle est une composition nutritionnelle synthétique.

**2.** Galacto-oligosaccharides ou composition nutritionnelle comprenant des galacto-oligosaccharides pour l'utilisation selon la revendication 1, où le contaminant alimentaire est une mycotoxine.

**3.** Galacto-oligosaccharides ou composition nutritionnelle comprenant des galacto-oligosaccharides pour l'utilisation selon la revendication 2, où la mycotoxine est le déoxynivalénol.

**4.** Galacto-oligosaccharides ou composition nutritionnelle comprenant des galacto-oligosaccharides pour l'utilisation selon l'une quelconque des revendications précédentes, où les galacto-oligosaccharides ou la composition nutritionnelle comprenant des galacto-oligosaccharides sont administrés à un nourrisson ou à un jeune enfant, de préférence à un nourrisson en cours de sevrage ou à un jeune enfant.

**5.** Galacto-oligosaccharides ou composition nutritionnelle comprenant des galacto-oligosaccharides pour l'utilisation selon l'une quelconque des revendications précédentes, où les galacto-oligosaccharides ou la composition nutritionnelle comprenant des galacto-oligosaccharides sont administrés à un sujet qui consomme des céréales ou des produits contenant des céréales.

**6.** Galacto-oligosaccharides ou composition nutritionnelle comprenant des galacto-oligosaccharides pour l'utilisation selon l'une quelconque des revendications précédentes, où l'administration ou la consommation se fait simultanément ou peu de temps avant ou après la consommation de céréales ou de produits contenant des céréales.

**7.** Composition nutritionnelle comprenant des galacto-oligosaccharides pour l'utilisation selon l'une quelconque des revendications précédentes, où la composition nutritionnelle comprend au moins 1 % en poids de galacto-oligosaccharides sur la base du poids sec de la composition nutritionnelle.

**8.** Composition nutritionnelle comprenant des galacto-oligosaccharides pour l'utilisation selon l'une quelconque des revendications précédentes, où la composition nutritionnelle est une formule pour nourrissons, une formule de suite ou une formule pour jeunes enfants, de préférence une formule de suite ou une formule pour jeunes enfants.

9. Bêta1,3'-galactosyllactose pour l'utilisation dans la prévention thérapeutique et/ou l'amélioration d'une diminution induite par un contaminant alimentaire de la réponse immunitaire adaptative des cellules B à la vaccination avec un antigène qui est dérivé d'un virus.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020229690 A
- WO 2013172714 A
- WO 2016013935 A
- EP 2440073 A
- US 2017209472 A
- US 2015164931 A
- FR 2723960
- EP 0778885 A
- WO 9606924 A

**Non-patent literature cited in the description**

- **VARASTEH et al.** *JPGN*, 2019, vol. 68 (1), 1049-1050
- **AKBARI et al.** *J Nutr*, 2015, vol. 145, 1604-1613
- **MARESCA et al.** The mycotoxin deoxynivalenol affects nutrient absorption in human intestinal epithelial cells. *J. Nutr.*, 2002, vol. 132, 2723-2731
- **AVANTAGGIATO et al.** Evaluation of the intestinal absorption of deoxynivalenol and nivalenol by an in vitro gastrointestinal model, and the binding efficacy of activated carbon and other absorbent materials. *Food and Chemical Toxicology*, 2004, vol. 42, 817-824
- **LEFORESTIER**. *Eur J Nutr*, 2009, vol. 48, 457-464
- **L. PROSKY et al.** *J. Assoc. Off. Anal. Chem.*, 1988, vol. 71, 1017-1023
- **XIAO et al.** *Front Immunol*, 2018, vol. 9 (452), https://doi.org/10.3389/fimmu.2018.00452